(19) Europäisches Patentamt
European Patent Office
Office européen des brevets

(11) **EP 0 825 170 B1**

(12) **EUROPÄISCHE PATENTSCHRIFT**

(45) Veröffentlichungstag und Bekanntmachung des Hinweises auf die Patenterteilung:
**10.01.2001   Patentblatt 2001/02**

(51) Int Cl.[7]: **C07C 59/01**, C07C 51/285

(21) Anmeldenummer: **97113444.0**

(22) Anmeldetag: **04.08.1997**

(54) **Verfahren zur Herstellung von Hydroxypivalinsäure**

Process for preparing hydroxypivalic acid

Procédé de préparation d'acide hydroxypivalique

(84) Benannte Vertragsstaaten:
**BE DE ES FR GB IT NL SE**

(30) Priorität: **16.08.1996   DE 19632922**

(43) Veröffentlichungstag der Anmeldung:
**25.02.1998   Patentblatt 1998/09**

(73) Patentinhaber: **BAYER AG**
**51368 Leverkusen (DE)**

(72) Erfinder:
• **Neumann, Karl-Heinz, Dr.**
 **53757 Sankt Augustin (DE)**
• **Joentgen, Winfried, Dr.**
 **51067 Köln (DE)**
• **Heitkamp, Dieter, Dr.**
 **51399 Burscheid (DE)**
• **Fiege, Helmut, Dr.**
 **51373 Leverkusen (DE)**

(56) Entgegenhaltungen:
• **H.P. FRANK ET AL.: "Über die Herstellung von Hydroxypivalinsaüre und deren Polyester" MONATSHEFTE FUR CHEMIE., Bd. 95, 1964, WIEN AT, Seiten 410-414, XP002054606**
• **J.H. PAYNE ET AL.: "The oxidation of aldehydes with hydrogen peroxide" JOURNAL OF THE AMERICAN CHEMICAL SOCIETY., Bd. 63, 1941, DC US, Seiten 226-228, XP002054607**
• **CHEMICAL ABSTRACTS, vol. 71, no. 7, 18.August 1969 Columbus, Ohio, US; abstract no. 30073j, HUANG, CHING-YUN ET AL.: "2,2-dimethyl-3-hydroxypropionic acid" Seite 248; Spalte 1; XP002054608 & JP 06 824 888 A (JAPAN GAS-CHEMICAL CO., INC.)**

**Beschreibung**

Die Erfindung betrifft ein Verfahren zur Herstellung von Hydroxypivalinsäure durch Oxidation von Hydroxypivalaldehyd mit Wasserstoffperoxid, bei dem das Oxidationsmittel zu einer wäßrigen Hydroxypivalaldehyd-Vorlage im Temperaturbereich von 60 bis 80°C so zudosiert wird, daß eine Konzentration an Wasserstoffperoxid von 4 Gew.-% im Reaktionsgemisch nicht überschritten wird und die Zugabe von Wasserstoffperoxid beendet wird, sobald die Hydroxypivalaldehyd-Konzentration im Reaktionsgemisch 1 Gew.-% unterschreitet.

Hydroxypivalinsäure ist ein wichtiges Ausgangsmaterial zur Herstellung von wasserbasierenden Lacksystemen, die zunehmend an Bedeutung gewinnen.

Hydroxypivalinsäure kann durch Kaliumpermanganatoxidation von Neopentylglykol oder durch Cannizzaro-Reaktion von Hydroxypivalaldehyd hergestellt werden. Beide Verfahren haben Nachteile. So ist die Ausbeute in der Oxidation von Neopentylglykol niedrig, während die Cannizzaro-Reaktion zu molaren Mengen an Neopentylglykol als Koppelprodukt führt. Deshalb sind beide Verfahren aus industrieller Sicht unattraktiv. Neben diesen beiden Herstellverfahren kann Hydroxypivalinsäure auch durch Luft/$O_2$-Oxidation von Neopentylglykol oder durch Wasserstoffperoxid-Oxidation von Hydroxypivalaldehyd erhalten werden. Die Oxidation von Neopentylglykol mit $O_2$ oder Luft an einem Pd/C-oder Pt/C-Katalysator in alkalisch wäßriger Lösung zu Hydroxypivalinsäure ist beispielsweise in JP 53/77 010 (1978) und in US 3.799.977 beschrieben. In US 3.799.977 fehlen Angaben zu Ausbeute und Selektivität völlig, und in JP 53/77 010 wird eine sehr zweifelhafte Ausbeute von 100 % angegeben, die lediglich auf dem Vergleich von IR-Banden des hergestellten Natrium-hydroxypivalats mit einem reinen Muster dieses Stoffes beruht. Da mit Neopentylglykol eine Ausgangsverbindung mit zwei völlig gleichen Hydroxylgruppen eingesetzt wird, weiß der Fachmann, daß bei der Oxidation Zwischenstufen und Überoxidationsprodukte entstehen, die nur mit großem Aufwand vom gewünschten Produkt abgetrennt werden können. Daneben entstehen infolge der Neutralisation des Reaktionsgemisches mindestens molare Mengen an NaCl oder $Na_2SO_4$, die entsorgt werden müssen. Dies alles spricht nicht für eine industrielle Herstellung von Hydroxypivalinsäure auf diesem Weg.

Weiterhin kann Hydroxypivalinsäure durch Oxidation von Hydroxypivalaldehyd mit Wasserstoffperoxid hergestellt werden. Dieses Verfahren wurde in Monatshefte für Chemie 95 (1964), 410 erstmals beschrieben und in einer überarbeiteten Variante in JP 43/24 888 (1968) erneut aufgegriffen. Im Verfahren nach Monatshefte wird, bezogen auf den Einsatz an Hydroxypivalaldehyd, eine Menge von 50 Mol-% einer 30 %igen wäßrigen Wasserstoffperoxidlösung zugegeben und der Reaktionsansatz 8 Stunden bei 50°C gerührt. Da in der Anfangsphase dieses Verfahrens mit einer hohen Konzentration an Wasserstoffperoxid gearbeitet wird, birgt eine solche Verfahrensvariante im technischen Maßstab große Sicherheitsrisiken. Weiterhin führt die niedrige Reaktionstemperatur von 50°C zu einer langen Reaktionszeit und damit zu einer schlechten Raum-Zeit-Ausbeute. Da sich $H_2O_2$ bei dieser Temperatur nur langsam zersetzt, kann nicht umgesetztes $H_2O_2$ auch in die Aufarbeitungsstufen geschleppt werden und dort eine Aufkonzentrierung erfahren. Nach einer mehrstufigen Aufarbeitung über das Na-Salz der Hydroxypivalinsäure wird in Monatshefte eine verunreinigte Hydroxypivalinsäure mit einem Schmelzbereich von 100 bis 122°C isoliert, während der Schmelzpunkt nach Literaturangaben 124 bis 126°C beträgt. Selbst wenn man die erhaltene rohe Hydroxypivalinsäure als rein betrachtet, beträgt die Ausbeute nur etwa 69 % der theoretischen Ausbeute, bezogen auf den Einsatz an Hydroxypivalaldehyd; der überaus große Schmelzpunktsbereich zeigt jedoch an, daß die Ausbeute an reinem Endprodukt deutlich niedriger ist.

JP 43/24 888 beschreibt eine Überarbeitung des Verfahrens nach Monatshefte unter Einsatz eines Katalysators, der in der Lage ist, $H_2O_2$ zu zersetzen. Als geeignete Katalysatoren werden sehr feine Teilchen aus Gold, Platin, Silber oder Glas sowie UV-Licht mit einer Wellenlänge von 200 bis 400 nm (2000 bis 4000 Å) angegeben. In den Ausführungsbeispielen wird jedoch nur der Einsatz von Ag-Pulver beschrieben. Die Oxidation von Hydroxypivalaldehyd erfolgt hierbei mit einem molaren $H_2O_2$-Überschuß von bis zu 20 % im Temperaturbereich von 80 bis 85°C. Im Unterschied zum Verfahren in Monatshefte wird die $H_2O_2$-Menge innerhalb einer Zeit von 1 bis 3 h zudosiert. In einem weiteren Beispiel in JP 43/24 888 wird die Oxidation des Hydroxypivalaldehyds ohne Katalysator, aber zweiphasig in Benzol/Wasser mit 30 %iger wäßriger $H_2O_2$-Lösung, die ebenfalls in 20 %igem molarem Überschuß in 3 h zudosiert wird, im Temperaturbereich von 58 bis 60°C durchgeführt. Auch dieses Verfahren hat somit etliche Nachteile. Zum einen ist der in den Beispielen eingesetzte Ag-Katalysator relativ teuer. Der Katalysator muß entweder durch Filtration oder durch Destillation des Produktes abgetrennt und wieder aufgearbeitet werden. Die Nacharbeitung der Ausführungsbeispiele von JP 43/24 888 zeigt zudem, daß nach beendeter $H_2O_2$-Oxidation in der Reaktionslösung nur 65 bis 68 % der theoretischen Ausbeute an Hydroxypivalinsäure, festgestellt mit Hilfe gaschromatografischer Analytik (G. C.) mit internem Standard, entstanden sind. JP 43/24 888 beschreibt ferner zur Reinigung der Pivalinsäure eine destillative Aufarbeitung, bei der in einer Nacharbeitung nur 28 % der ursprünglich in der wäßrigen Oxidationslösung vorhandenen Hydroxypivalinsäure bei 156 bis 159°C/33 mbar abdestilliert werden konnten. Neben einer Verteuerung des Verfahrens durch den Einsatz eines Überschusses an $H_2O_2$ birgt dieser wiederum die bereits oben erwähnten Sicherheitsrisiken, insbesondere wenn zusätzlich ein leicht brennbares Lösungsmittel wie Benzol eingesetzt wird. Weiterhin entsteht bei der $H_2O_2$-Oxidation von Hydroxypivalaldehyd zu Hydroxypivalinsäure ein brennbares Abgas, das sich, abhängig vom Reaktionsfortschritt aus unterschiedlichen Mengen an $H_2$, $O_2$, $CO$ und $CO_2$ zusammensetzt; bei einer Überschußdosierung von $H_2O_2$ wird diese Abgassituation weiter verschärft. Schließlich erhält man bei der Dosierung von überschüssigem $H_2O_2$ gegen Ende der Reaktion höhere Konzentrationen an Peroxid, die bei der Übertragung in den technischen Maßstab aus Sicherheitsgründen nicht zu vertreten sind.

Andere Katalysatoren, die zur Oxidation von aliphatischen Aldehyden mit $H_2O_2$ vorgeschlagen wurden, wie beispielsweise $(NH_4)_6Mo_7O_{24}$ x 4 $H_2O$ oder $CeCl_2$ x 7 $H_2O$ (Tetrahedron Letters 25 (1984), 173-176; Israel J. of Chem. 24 (1984), 134-143) bereiten ebenfalls Probleme bei der Isolierung von Hydroxypivalinsäure, wenn sie auf die Oxidation von Hydroxypivalaldehyd angewendet werden. Durch diese Katalysatoren entstehen gelbe Lösungen, die vor der Isolierung durch Zugabe von Aktivkohle aufwendig entfärbt werden müssen.

Es bestand daher weiterhin das Bedürfnis, ein Verfahren zur $H_2O_2$-Oxidation von Hydroxypivalaldehyd zu Hydroxypivalinsäure zur Verfügung zu haben, welches die aufgeführten Nachteile vermeidet. Erfindungsgemäß wird ein solches Verfahren bereitgestellt, bei dem

a) wäßriges $H_2O_2$ umsatzabhängig so zur wäßrigen Vorlage von Hydroxypivalaldehyd zudosiert wird, daß eine Konzentration an $H_2O_2$ von 4 Gew.-% im gesamten Reaktionsansatz nicht überschritten wird,

b) die Oxidationsreaktion bei 60 bis 80°C durchgeführt wird und

c) die Zugabe von $H_2O_2$ beendet wird, wenn die Konzentration an Hydroxypivalaldehyd in der Vorlage unter 1 Gew.-% der Gesamtmenge an Reaktionsgemisch sinkt.

Die Erfindung betrifft demnach ein Verfahren zur Herstellung von Hydroxypivalinsäure durch Oxidation von Hydroxypivalaldehyd mit Wasserstoffperoxid, das dadurch gekennzeichnet ist, daß bei 60 bis 80°C eine wäßrige Wasserstoffperoxidlösung zu einer wäßrigen Hydroxypivalaldehyd-Vorlage so zudosiert wird, daß die Konzentration an Wasserstoffperoxid 4 Gew.-% des Gesamtgewichts des Reaktionsgemisches nicht überschreitet und die Zugabe von Wasserstoffperoxid beendet wird, wenn die Konzentration an Hydroxypivalaldehyd 1 Gew.-% des Gesamtgewichts des Reaktionsgemisches unterschreitet.

Es wird angenommen, daß das erfindungsgemäße Verfahren in Übereinstimmung mit folgender Reaktionsgleichung steht:

$$HO\text{-}CH_2\text{-}C(CH_3)_2\text{-}CHO + H_2O_2 \rightarrow$$

$$HO\text{-}CH_2\text{-}C(CH_3)_2\text{-}COOH + H_2O$$

(zum Mechanismus siehe z.B.: J. Am. Chem. Soc. 63, (1941), S. 226).

Der zum Einsatz kommende Hydroxypivalaldehyd kann, wie vielfach beschrieben ist, durch basenkatalysierte Kondensation von Isobutyraldehyd mit Formaldehyd hergestellt werden. Als Basen kommen beispielsweise Trialkylamine (DE-OS 19 57 591), $Na_2CO_3$ (JP 43/24 888) oder (Erd)alkalimetallhydroxide (Chem. Ber. 102, (1969), 1606) in Frage. Hydroxypivalaldehyd wird im erfindungsgemäßen Verfahren in wäßriger Lösung, die bei höheren Konzentrationen als Schmelze bei schwach erhöhter Temperatur vorliegt, eingesetzt; die Konzentration an Pivalaldehyd beträgt hierin 40 bis 80 Gew.-%, bevorzugt 50 bis 70 Gew.-%, bezogen auf die Gesamtmenge dieser Lösung bzw. Schmelze.

$H_2O_2$ als Oxidationsmittel wird in wäßriger Lösung mit einer $H_2O_2$-Konzentration von 5 bis 70 Gew.-%, bevorzugt 20 bis 50 Gew.-% $H_2O_2$ in der Gesamtlösung eingesetzt. $H_2O_2$ wird im erfindungsgemäßen Verfahren in Abhängigkeit von seinem Verbrauch so dosiert, daß seine Konzentration im Reaktionsgemisch 4 Gew.-%, bevorzugt 2,5 Gew.-%, bezogen auf das Gesamtgewicht des Reaktionsgemisches, nicht überschreitet. Die Zugabe von $H_2O_2$ wird hierbei ständig, beispielsweise durch jodometrische Titration und durch gaschromatografische Analyse (G.C.) mit innerem Standard des noch vorhandenen Hydroxypivalaldehyds, begleitet und wird unterbrochen, wenn die Konzentration an Hydroxypivalaldehyd 1 Gew.-% unterschreitet, also der Hydroxypivalaldehyd in einer restlichen Menge von 0,05 bis 0,99 Gew.-%, bevorzugt 0,2 bis 0,95 Gew.-%, bezogen auf das Gesamtgewicht des Reaktionsgemisches vorliegt. Hierbei wurde beobachtet, daß etwa 75 bis 85 Mol-% $H_2O_2$, bezogen auf die Molmenge an eingesetztem Hydroxypivalaldehyd, benötigt wird. In Abhängigkeit von der Größe des Reaktionsgefäßes und offenbar abhängig von der spezifischen Wandfläche pro Volumeneinheit Reaktionsgemisch gibt es innerhalb dieses Bereiches geringe Schwankungen. So werden bei kleineren Ansätzen $H_2O_2$-Verbräuche im oberen Teil des angegebenen Bereiches (etwa 80 bis 85 Mol-% $H_2O_2$) verbraucht, während in technischen Reaktoren vorteilhafterweise Verbräuche von herunter bis zu 75 Mol-% $H_2O_2$ beobachtet wurden. Die Ausbeute an Hydroxypivalinsäure, bezogen auf den Einsatz an Hydroxypivalaldehyd, beträgt etwa 72 bis 76 % der Theorie in Lösung. Es wurde weiterhin beobachtet, daß eine größere $H_2O_2$-Menge als die oben angegebene zu einer verschlechterten Selektivität an Hydroxypivalinsäure führt; dies geht mit einer

erhöhten CO$_2$-Emission einher, was für einen weitergehenden oxidativen Abbau der Hydroxypivalinsäure spricht.

Das erfindungsgemäße Verfahren wird im Bereich von 60 bis 80°C, bevorzugt im Bereich von 63 bis 78°C durchgeführt.

Das erfindungsgemäße Verfahren hat wirtschaftliche Vorteile bezüglich seiner Handhabung, seiner Sicherheitsaspekte und seiner Selektivitäten. So hat ein katalysatorfreies Oxidationsverfahren den Vorteil, daß mindestens ein Aufarbeitungsschritt zur Abtrennung des Katalysators (Filtration, Zentrifugieren) und zur Entfernung von etwa vorhandenen färbenden löslichen Katalysatoren (Aktivkohlebehandlung) entfällt. Weiterhin wurde die vorteilhafte Temperaturführung im Bereich von 60 bis 80°C aufgefunden, während im Verfahren nach Monatshefte offenbar aus Sicherheitsgründen eine Temperatur von 50°C eingehalten wird; dadurch kann die Dosierzeit von H$_2$O$_2$ auf etwa 3 bis 4 h gesenkt werden, während im Verfahren nach Monatshefte 8 h Reaktionszeit benötigt werden, was eine sehr geringe Raum-Zeit-Ausbeute ergibt. Es wurde weiterhin beobachtet, daß beim Arbeiten bei Temperaturen oberhalb von 80°C unabhängig vom Einsatz eines Katalysators bereits eine merkliche H$_2$O$_2$-Zersetzung eintritt; dies kann wie in JP 43/24 888 nur durch einen deutlich höheren Einsatz an H$_2$O$_2$ kompensiert werden, wodurch aber das gesamte Verfahren unnötig verteuert wird. Das erfindungsgemäße Verfahren ist somit erstaunlich vorteilhaft sowohl bezüglich der erforderlichen H$_2$O$_2$-Menge als auch bezüglich der erzielbaren Selektivität an Hydroxypivalinsäure.

**Beispiel 1** (Hydroxypivalaldehyd HPA)

In einem 4 l-Vierhalskolben mit Rührer, Innenthermometer, Intensivkühler und Tropftrichter wurden 1745,8 g 35 %ige Formalinlösung (20,35 Mol) und 101,0 g Triethylamin (1,0 Mol) vorgelegt und unter einer N$_2$-Atmosphäre auf 55°C erwärmt. Nach Erreichen dieser Temperatur tropfte man in 45 min. 1456,8 g 99 %igen Isobutyraldehyd (20,0 Mol) zu. Die Reaktion war exotherm; um die Temperatur von 55°C zu halten, mußte man gut kühlen. Nach beendeter Isobutyraldehyzugabe wurde die Reaktionsmischung in 1 h von 55°C auf 90°C erwärmt. Man ließ danach wieder abkühlen und tauschte den Intensivkühler gegen eine Destillationseinrichtung mit einem 500 ml Vorlagekolben aus. Zur Entfernung von nicht umgesetztem Isobutyraldehyd strippte man bei 55 bis 57°C Sumpftemperatur / 175 bis 185 mbar insgesamt 388,7 g Destillat ab. Man erhielt als Rückstand 2820 g wäßriger HPA-Lösung mit einem Gehalt von 64,78 % (G.C. int. Std.) ≙ 17,9 Mol ≙ 89,5 % der theoretischen Ausbeute.

**Beispiel 2** (Oxidation von HPA mit H$_2$O$_2$ zu Hydroxypivalinsäure)

Während der Oxidation wurden der HPA-Umsatz mittels G.C.-Analysen und die H$_2$O$_2$-Konzentration durch iodometrische Titration begleitend bestimmt. In einem 2 l-Vierhalskolben mit Rührer, Rückflußkühler, Tropftrichter, Innenthermometer, pH-Elektrode und Redox-Elektrode wurden 574 g einer aufgeschmolzenen, wäßrigen, 3,39 Mol HPA enthaltenden Lösung vorgelegt und auf 70°C erwärmt. Bei dieser Temperatur dosierte man innerhalb von 3 h 222 ml 35 %ige wäßrige H$_2$O$_2$-Lösung (2,58 Mol) so ein, daß eine Peroxidkonzentration von 2,5 Gew.-% nicht überschritten wurde. Nach beendeter Dosierung wurde die HPA-Restkonzentration durch eine G.C.-Analyse bestimmt. Mit dem Ziel, die HPA-Konzentration unter 1 Gew.-% zu drücken, wurden im vorliegenden Beispiel weitere 14,5 ml 35 %ige H$_2$O$_2$-Lösung in 20 min. eindosiert. Die Gesamtmenge an H$_2$O$_2$ betrug also 236,5 ml ≙ 267,2 g ≙ 93,54 g H$_2$O$_2$ ≙ 2,75 Mol. Während der H$_2$O$_2$-Dosierung entstanden ca. 21 l eines brennbaren Abgases, welches sich umsatzabhängig aus unterschiedlichen Mengen H$_2$, O$_2$, CO, CO$_2$ und flüchtigen organischen Verbindungen zusammensetzte. Nach beendeter H$_2$O$_2$-Dosierung wurde noch 1 h bei 70°C nachgerührt, man ließ abkühlen und prüfte den HPA-Gehalt der Lösung mittels G.C. mit int. Std. Ausbeute: 795,6 g wäßrige Lösung, HPA-Gehalt: 37,22 % (G.C.) ≙ 296,1 g Hydroxypivalinsäure = 2,507 Mol ≙ 74,0 % der theoretischen Ausbeute, bezogen auf den HPA-Einsatz.

**Beispiel 3** (zum Vergleich)

In der in Beispiel 2 beschriebenen Apparatur wurden 726,2 g einer wäßrigen Lösung, die 3,035 Mol rohen HPA enthielt, vorgelegt und auf 60°C erwärmt. Innerhalb von 90 min. tropfte man 349,9 g (3,59 Mol) einer 35 %igen wäßrigen H$_2$O$_2$-Lösung in die 60 bis 70°C warme Vorlage. Man rührte danach noch 30 min. bei gleicher Temperatur nach und verkochte anschließend in 4 h das überschüssige H$_2$O$_2$. Zur entgültigen Entfernung restlicher Peroxide wurden noch 18,5 ml 39 %iger Natriumhydrogensulfitlösung zugegeben. Die Reaktionslösung wurde dann mit 96 %iger H$_2$SO$_4$ auf pH = 2,5 gestellt. Mittels G.C.-Analytik (int. Std.) konnten insgesamt 1,66 Mol Hydroxypivalinsäure ≙ 54,7 % der theoretischen Ausbeute nachgewiesen werden. Hierdurch zeigte sich, daß der Einsatz eines H$_2$O$_2$-Überschusses (HPA: H$_2$O$_2$ ~ 1:1,2 Mol) und eine schnelle Dosierung die zu höheren H$_2$O$_2$-Konzentrationen im Ansatz führt, keinen Vorteil bringt, sondern die Hydroxypivalinsäure wird sogar durch Überoxidation abgebaut.

**Beispiel 4** (zum Vergleich)

In der in Beispiel 2 beschriebenen Apparatur wurden 555 g einer aufgeschmolzenen wäßrigen, 3,51 Mol HPA enthaltenden Lösung, die analog Beispiel 1 hergestellt worden war, vorgelegt. Man gab 350 mg Silberpulver zur Vorlage und erwärmte auf 80°C. Bei dieser Temperatur wurden in 150 min (incl. Unterbrechungen für Peroxidtitrationen) 400 g 32 %ige wäßrige $H_2O_2$-Lösung $\triangle$ 4,25 Mol $H_2O_2$ zugetropft. Nach beendeter Zugabe rührte man noch 30 min. bei 80°C. Anschließend ließ man abkühlen, filtrierte den Silberkatalysator ab und wusch den Katalysator mit Wasser nach. Man erhielt 917,6 g einer wäßrigen Lösung mit einem Hydroxypivalinsäuregehalt von 29,85 % (G.C. int. Std.) = 273,9 g = 2,32 Mol $\triangle$ 66,1 % der theoretischen Ausbeute.

## Patentansprüche

1. Verfahren zur Herstellung von Hydroxypivalinsäure durch Oxidation von Hydroxypivalaldehyd mit Wasserstoffperoxid, dadurch gekennzeichnet, daß bei 60 bis 80°C eine wäßrige Wasserstoffperoxidlösung zu einer wäßrigen Vorlage von Hydroxypivalaldehyd so zudosiert wird, daß die Konzentration an Wasserstoffperoxid 4 Gew.-% des Gesamtgewichts des Reaktionsgemisches nicht überschreitet und die Zugabe von Wasserstoffperoxid beendet wird, wenn die Konzentration an Hydroxypivalaldehyd 1 Gew.-% des Gesamtgewichts des Reaktionsgemisches unterschreitet.

2. Verfahren nach Anspruch 1, dadurch gekennzeichnet, daß der Hydroxypivalaldehyd als wäßrige Lösung oder Schmelze mit einem Gehalt von 40 bis 80 Gew.-%, bevorzugt 50 bis 70 Gew.-%, bezogen auf die Lösung bzw. Schmelze eingesetzt wird.

3. Verfahren nach Anspruch 1, dadurch gekennzeichnet, daß die Konzentration an Wasserstoffperoxid 2,5 Gew.-% des Gesamtgewichts des Reaktionsgemisches nicht überschreitet.

## Claims

1. Process for the preparation of hydroxypivalic acid by oxidation of hydroxypivalaldehyde with hydrogen peroxide, characterized in that an aqueous hydrogen peroxide solution is metered into an aqueous reservoir of hydroxypivalaldehyde at 60 to 80°C such that the concentration of hydrogen peroxide does not exceed 4% by weight of the total weight of the reaction mixture, and the addition of hydrogen peroxide is ended when the concentration of hydroxypivalaldehyde falls below 1% by weight of the total weight of the reaction mixture.

2. Process according to Claim 1, characterized in that the hydroxypivalaldehyde is employed as an aqueous solution or melt having a content of 40 to 80% by weight, preferably 50 to 70% by weight, based on the solution or melt.

3. Process according to Claim 1, characterized in that the concentration of hydrogen peroxide does not exceed 2.5% by weight of the total weight of the reaction mixture.

## Revendications

1. Procédé pour la préparation de l'acide hydroxypivalique par oxydation de l'aldéhyde hydroxypivalique à l'aide du peroxyde d'hydrogène, caractérisé en ce que, à de l'aldéhyde hydroxypivalique aqueux, on ajoute entre 60 et 80°C une solution aqueuse de peroxyde d'hydrogène en veillant à ce que la concentration en peroxyde d'hydrogène ne dépasse pas 4 % du poids total du mélange de réaction et on arrête l'addition du peroxyde d'hydrogène lorsque la concentration en aldéhyde hydroxypivalique est tombée au-dessous de 1 % du poids total du mélange de réaction.

2. Procédé selon la revendication 1, caractérisé en ce que l'on met en oeuvre l'aldéhyde hydroxypivalique à l'état de solution aqueuse ou de masse aqueuse à une teneur de 40 à 80 % en poids, de préférence de 50 à 70 % en poids de la solution ou masse totale.

3. Procédé selon la revendication 1, caractérisé en ce que la concentration en peroxyde d'hydrogène ne dépasse pas 2,5 % du poids total du mélange de réaction.